# EUROPEAN PATENT SPECIFICATION

(11) **EP 3 581 174 B1**
(45) Date of publication and mention of the grant of the patent: **25.03.2026**
(21) Application number: 18750777.7
(22) Date of filing: 08.02.2018
(51) Int. Cl.: A61K 9/50, A61K 9/19, A61K 47/46, A61K 36/18, A23L 21/20, A23L 27/00

(54) **PURIFIED POLLEN PARTICLES AND USE THEREOF FOR ADMINISTERING NANOSYSTEMS**
GEREINIGTE POLLENPARTIKEL UND DEREN VERWENDUNG ZUR VERABREICHUNG VON NANOSYSTEMEN
PARTICULES PURIFIÉES DE POLLEN ET LEURS UTILISATION POUR ADMINISTRER DES NANOSYSTÈMES

(30) Priority: 09.02.2017 ES 201730151; 09.02.2017 ES 201730152
(43) Date of publication of application: 18.12.2019
(73) Proprietor: Universidade de Santiago de Compostela, 15782 Santiago de Compostela (ES)
(72) Inventor: CSABA, Noemi, 15782 Santiago de Compostela (ES); PAN DELGADO, Diego, 15782 Santiago de Compostela (ES); REIMONDEZ TROITIÑO, Sonia, 15782 Santiago de Compostela (ES); GARCIA FUENTES, Marcos, 15782 Santiago de Compostela (ES)
(74) Representative: ABG Intellectual Property Law, S.L.
(86) International application number: PCT/ES2018/070092
(87) International publication number: WO 2018/146365

(56) References cited:
- WO-A1-2009/077749
- WO-A1-2017/010945
- WO-A1-2017/010945
- US-A- 5 013 552
- LIN, HAISHENG ET AL.: "POLLENKITT WETTING MECHANISM ENABLES SPECIES-SPECIFIC TUNABLE POLLEN ADHESION", LANGMUIR, vol. 29, no. 9, 2013, pages 3012 - 3023, XP055532357
- MUNDARGI, RAGHAVENDRA C. ET AL.: "SUNFLOWER POLLEN AS A DRUG DELIVERY VEHICLE", SMALL, vol. 12, no. 9, 2016, pages 1167 - 1173, XP055351867

## Description

### Field of the Invention

The present disclosure relates to purified pollen particles, to their manufacturing methods, and to uses thereof in the administration of encapsulated active ingredients.

### State of the Art

Transmucosal administration of active ingredients, for example drugs, is one of the most accepted routes by patients. It includes, for example, nasal, oral (for example, absorption through the small or large intestine), or ocular administration.

The transmucosal drug route of administration has certain limitations inherent to its physicochemical characteristics. In this sense, the most widely used route of administration is the parenteral route which, however, has its drawbacks. For example, the preparation of injectables requires sterile production conditions, their handling usually involves medical personnel, is more uncomfortable for the patient, and involves greater risks of infection caused by improper use of needles. Therefore, transmucosal administration is often an appealing alternative.

One of the most common delivery routes is oral administration. This is also true in the field of nutrition, where dosage forms for different additives and nutrients are constantly sought to improve currently existing technologies.

The epithelium of human intestine is highly absorbent and made up of a large number of microvilli with a total absorption surface of up to 350 m², which is ideal for efficient absorption. However, said pathway is not without problems. Many macromolecular components exhibit low permeability and poor stability due to the aggressive environment of the digestive tract. In addition to being stable in this medium, they must overcome the mucosal barrier in order to reach their target site and be absorbed before their elimination.

Intestinal mucus is a complex hydrogel comprising proteins, carbohydrates, lipids, salts, and other components. It is continuously secreted and renewed to prevent the entry of pathogenic agents, to lubricate and protect the digestive tract, but at the same time it allows the passage of nutrients. Due to the presence of this intestinal mucosal barrier and its dynamic nature, orally administered particles may not have a suitable residence time for their absorption and may be eliminated without fulfilling their purpose. Mucoadhesive polymers such as some cellulose, polyacrylate, starch, or chitosan derivatives have been used for improving particle residence time in the intestinal tract. However, most of the mucoadhesive polymer-based nanosystems developed up until now tend to remain anchored in the less adherent mucus layer without penetrating the intestinal mucosal barrier, thereby being exposed to rapid elimination.

When nanoparticles are used, oral administration can be considered a complex, multistep process. The nanoparticles must first reach the intestinal mucosa without degrading and without releasing the active ingredient prematurely. Then, they must adhere to the outermost mucosa. This adhesion must be strong enough to prevent their rapid elimination, but at the same time should allow their penetration into deeper mucosal layers. Once the deepest layers of the mucosa are reached, the nanoparticles must release the active ingredient with the required release profile.

To achieve a suitable administration, the delivery of therapeutic drugs through the digestive tract has been evaluated by means of physical methods (Traverso G, Schoellhammer CM, Schroeder A, Maa R, Lauwers GY, Polat BE, Anderson DG, Blankschtein D, Langer R J Pharm Sci 2015, 104, 362-367). For example, the robotic pill is one of the most recent platforms for the oral delivery of large molecules. Although this strategy is promising, it still involves the perforation and alteration of the mucosa, which is particularly concerning in the case of chronic diseases which requires repeated administrations for a long term, sometimes for years.

Moreover, efforts have been made in using spores or pollen as vehicles for transporting molecules. Spores are produced by lower plants or cryptogams, also known as sporophytes. Pollen is produced by seed plants (spermatophytes) and contains a microgametophyte (male gametophyte). The structure and composition of spores and pollen vary by species. A pollen or spore particle is essentially made up of genetic material contained in a cytoplasm, which is coated by a first inner layer referred to as intine in the case of pollen and endospore in the case of spores. These are in turn coated with a second layer called exine and exospore, respectively. The composition of the cytoplasm, intine, and endospore varies.

Various papers based on the use of spores as vehicles for the administration of molecules can be found in the literature.

Several documents published by the UNIVERSITY OF HULL describe the use of *Lycopodium clavatum* (pteridophytes) spores for the encapsulation of low molecular weight drugs and oils. For example, the document WO2005000280 describes lipid layer-free, hollow spores, i.e., spores stripped of their cytoplasm and intine, prepared from *Lycopodium clavatum,* which are loaded with dietetic or pharmaceutical ingredients and have a protein content below 0.5%. These hollow spores are prepared through a complex process of aggressive washing with acidic, basic, and organic media. This document does not indicate the possibility of using the hollow spores as carriers for nanosystems. Document WO2006064227 describes the use of this same technology based on *Lycopodium clavatum* spores for the creation of magnetic formulations. Other applications belonging to the same authors describe compositions containing hollow *Lycopodium clavatum* spores following the same principles: documents WO2007012856 (antioxidant activity), WO2007012857 (topical compositions), WO2009077749 (compositions comprising protective additives), and WO2010004334 (improved whitening). None of the cases mentions the possibility of incorporating nanosystems to the hollow spores, and they refer at all times to a hollow exine capsule of *Lycopodium clavatum* spores that is free of the outer lipid layer and prepared by a complex sequence of acidic and basic washes, among others. These hollow spores have a limited capacity for controlling the release profile, showing highly variable release times. Furthermore, Lorch, M., et al. Chem. Comm., 2009, 6442-6444 have described the behavior of these spores in blood plasma and observed that they are unstable and collapse in said biological medium.

Following essentially the same methods, other research groups have developed technologies related to hollow *Lycopodium clavatum* spores, for example, as antigen carriers for oral vaccines (Shashwti U. Atwe, Yunzhe Ma, Harvinder Singh Gill, Journal of Controlled Release, 2014, 194, 45-52).

Shwan A. Hamad, Amro F. K. Dyab, Simeon D. Stoyanovb, Vesselin N. Paunov J. Mater. Chem., 2011, 21, 18018-18023 describe a method for encapsulating a combination of cells and magnetic nanoparticles within hollow *Lycopodium clavatum* spores. Said method comprises breaking up the spores by means of compression and then performing incubation in the presence of a mixture of cells and magnetic nanoparticles. Despite contemplating the possibility of incorporating inorganic nanoparticles, the method described in this document makes necessary to break up the spore, compromising its physicochemical properties and therefore its usefulness as an administration vehicle in biological systems.

The surface of the exine of pollen particles has an additional complex lipid layer that is a mixture of proteins, lipids, and other molecules (known as *"pollenkitt"*). The intine is generally formed by cellulose, whereas the exine is made up of a proteic material called sporopollenin, the exact composition of which is unknown. The exine is an extremely tough layer that is stable under acidic and basic conditions and has a high porosity. As a result of these properties, various technologies which allow isolating the exine, i.e., stripping the intine and genetic material from inside the exine, as well as cleaning the outer surface of the lipid layer or *pollenkitt,* have been tested. Once isolated, their use as a delivery systems for the administration of the molecules of interest is tested.

Raghavendra C. Mundargi, Michael G. Potroz, Soohyun Park, Hitomi Shirahama, Jae Ho Lee, Jeongeun Seo, Nam-Joon Cho, small 2016, 12, No. 9, 1167-1173 describe the use of *Helianthus annuus* (sunflower) pollen particles previously washed with ethyl ether for encapsulating BSA, and suggest the possibility of using different pollen spores for encapsulating small molecules, proteins, peptides, growth factors, or biosimilars. BSA loading comprises incubation or incubation under vacuum. The preparation method described in this document does not include washing with aqueous solutions.

A singular example can be found in W. Brandon Goodwin, Ismael J. Gomez, Yunnan Fang, J. Carson Meredith, Kenneth H. Sandhage, Chem. Mater. 2013, 25, 4529-4536, in which *Helianthus annuus* (sunflower) pollen particles are washed with a mixture of chloroform and methanol for subsequent use as templates in the preparation of replicas based on iron oxide. The resulting product is subjected to pyrolysis, remaining only the iron oxide "shell" with the shape of the original pollen particle. Additionally, WO 2017/010945 A1 discloses the use of whole pollen spores (comprising the exine layer and the intine layer with the cytoplasmic organelles therein) for the encapsulation of bovine serum albumin.

Studies focused on studying the composition of pollen particles can also be found in the literature, although they do not describe possible uses thereof. Some of these studies can be found, for example, in Doughty, J.; Hedderson, F.; McCubbin, A.; Dickinson, H. Proc. Natl. Acad. Sci., 1993, 90, 467-471, in which *Brassica oleracea* spores are washed with cyclohexane for studying the coating proteins thus extracted. Mohamed Elfatih H. Bashir, Jason M. Ward, Matthew Cummings, Eltayeb E. Karrar, Michael Root, Abu Bekr A. Mohamed, Robert M. Naclerio, Daphne Preuss, PLoS ONE 8(1): e53337 study pollen coat proteins of grasses from the *Poaceae* family, including, *Cynodon dactylon* (Bermuda), *Phleum protense* (Timothy), *Poa pratensis,* or *Dactylis glomerata.* In this document, the pollen is washed with cyclohexane for extracting lipophilic proteins from its surface. It is reported that, as a result of treatment, the microchannels of the exine break down, the pollen particle thereby losing its structure.

The use of pollen is not without problems. Although pollen can have similar sizes, its morphology and biocompatibility are not assured once treated, particularly in view of the aggressiveness of some of the treatments to which they are subjected. Furthermore, there is a need to consider if this treatment will keep its morphology intact and if it will be able to incorporate and subsequently protect and release the products that can be incorporated, for example, nanosystems.

Nasal and ocular administrations face problems similar to those discussed above for the case of oral administration, and delivery of active ingredients through these routes such that they have a suitable stability and residence time remains a challenge.

Therefore, it would be desirable to find alternative methods for the administration of nanosystems that can overcome the limitations indicated above and can be obtained by means of simple methods.

### Brief Description of the Invention

Any references in the description to methods of treatment refers to the compounds, pharmaceutical compositions and medicament of the present invention for use in a method for treatment of the human (or animal) body by therapy (or for diagnosis).

The authors of the present disclosure now provide a delivery system which solves the problems set forth in the state of the art. Said system is based on purified pollen particles. They are obtained by means of a pollen purification method which improves pollen biocompatibility and allows the reproducible incorporation of nanosystems.

In that sense, a first aspect of the invention relates to a delivery system comprising:
- a purified pollen particle comprising an intine layer and an exine layer; wherein said purified pollen particle does not comprise a lipid layer (*pollenkitt*); and
- a nanosystem; wherein the nanosystem is a lyophobic colloidal particle with a mean particle diameter comprised between 1 nm and 500 nm, wherein said mean diameter is measured by means of photon correlation spectroscopy.

Although hollow spores have been used in other studies as containers for molecules, neither the possibility of eliminating the lipid layer nor the possible use thereof as an administration platform for nanosystems, and particularly for nanosystems through mucosal routes, has been reported. Furthermore, researchers have found in both *ex vivo* and *in vivo* experiments that the simple purification method used results in purified pollen particles with a surprising adhesion capacity, and most fundamentally, intestinal mucosa penetration capacity, even after the surface incorporation and/or absorption of nanosystems, which significantly improves current knowledge.

The delivery system of the invention can be incorporated into different compositions, which constitutes an additional aspect of the invention. An additional aspect is related to the compositions of the invention for use as a medicament. This opens up the possibility of a new platform for the use thereof in manufacturing medicinal products for the treatment of diseases or medical conditions such as, for example, metabolic, immune, gastrointestinal, cardiovascular, joint, rare, tropical, oncological diseases, among others, and furthermore for the prevention of infectious diseases, such as vaccines, and preferably in the case of chronic and/or metabolic diseases requiring repeated administrations, particularly through mucosal routes. Additional aspects also relate to the use thereof in manufacturing a medicinal product for the controlled release of a pharmaceutically acceptable active ingredient. Given their stability *in vivo* and excellent intestinal mucosa adhesion, the delivery systems of the invention also have an excellent use as a food supplement in the administration of a dietetically acceptable ingredient, and for the use thereof in manufacturing a medicinal product for oral, nasal, or ocular administration, including the possibility of designing treatments through these routes which were previously impossible.

Not only have excellent stability and adhesion results been obtained. Furthermore, the purified pollen particles are obtained by means of very simple methods from pollen that can be obtained at affordable prices and in viable amounts for industrial application. Therefore, an additional aspect of the invention relates to a method for preparing the delivery system according to the first aspect of the invention, which method comprises the steps of
(a) providing
   - a pollen particle; and
   - a nanosystem; wherein the nanosystem is a lyophobic colloidal particle with a mean particle diameter comprised between 1 nm and 500 nm, wherein said mean diameter is measured by means of photon correlation spectroscopy; and wherein the nanosystem is preferably an organic nanosystem;
(b) washing the pollen particle in an aqueous medium; preferably wherein the aqueous medium is a buffered aqueous medium at a pH comprised between 4.5 and 9;
(c) washing the pollen particle with an organic solvent; wherein step (c) can be performed before or after step (b); to obtain a purified pollen particle comprising the intine layer and the exine layer; wherein the purified pollen particle does not comprises a lipid layer (*pollenkitt*); and
(d) contacting the purified pollen particle with the nanosystem to obtain a delivery system.

Unlike processes disclosed in state of the art, these simple processes prevent contacting the pollen particles with strong basic and/or acidic media and comprise several aggressive washes with different acids and bases (see document WO2005000280, for example). With the method of the invention, the intine is partially or completely retained, and the resulting purified pollen particle is more stable in biological fluids (see the examples below) than the hollow spores that only retain the exine layer.

Although hollow spores have been used in other studies as containers for molecules, neither the possibility of retaining both the exine and the intine, nor the possible use thereof as an administration platform for nanosystems, and particularly for nanosystems through mucosal routes, has been reported. Furthermore, researchers have found in both *ex vivo* and *in vivo* experiments that the simple purification methods used result in purified pollen particles with a surprising adhesion capacity, and most fundamentally intestinal mucosa penetration capacity, even after the surface incorporation and/or absorption of nanosystems, which significantly improves current knowledge.

The delivery system of the invention can be incorporated into different compositions, which constitutes an additional aspect of the invention. An additional aspect also relates to the possibility of using same as a medicinal product. This opens up the possibility of a new platform for the use thereof in manufacturing medicinal products for the treatment of diseases or medical conditions such as, for example, metabolic, immune, gastrointestinal, cardiovascular, joint, rare, tropical, oncological diseases, among others, and furthermore for the prevention of infectious diseases, such as vaccines, preferably in the case of chronic and/or metabolic diseases requiring repeated administrations, particularly through mucosal routes. Additional aspects also relate to the use thereof in manufacturing a medicinal product for the controlled release of a pharmaceutically acceptable active ingredient. Given their stability *in vivo* and excellent intestinal mucosa adhesion, the delivery system of the invention also have an excellent use as a food supplement in the administration of a dietetically acceptable ingredient, and for the use thereof in manufacturing a medicinal product for oral, nasal, or ocular administration, including the possibility of designing treatments through these routes which were previously impossible.

The purified pollen particles obtained using any of the described washing methods can incorporate nanosystems by means of a simple incubation process.. An additional aspect of the invention relates to a composition comprising a nanosystem and a purified pollen particle, preferably spinose pollen, comprising an intine layer and an exine layer, but the lipid layer (*pollenkitt*) of which has been eliminated.

The purified pollen particles herein described provide a new strategy for the administration, particularly for transmucosal administration, of nanosystems. They allow simultaneously administering a large number of nanosystems and improve their mucosal adhesion and penetration possibilities.

### Brief Description of the Drawings

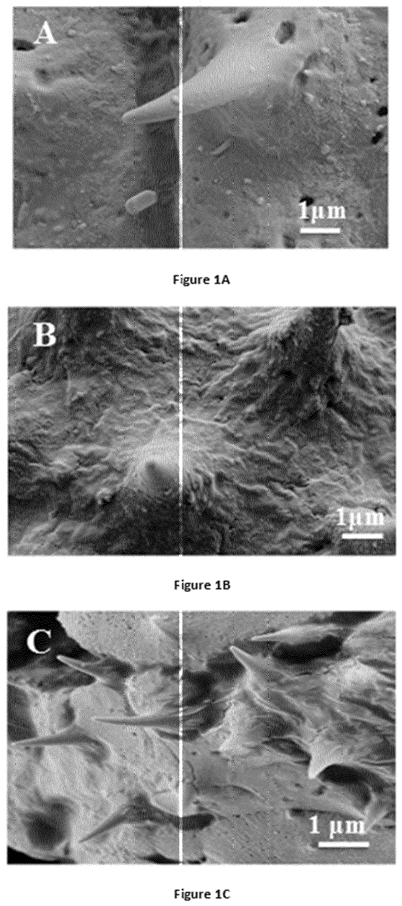

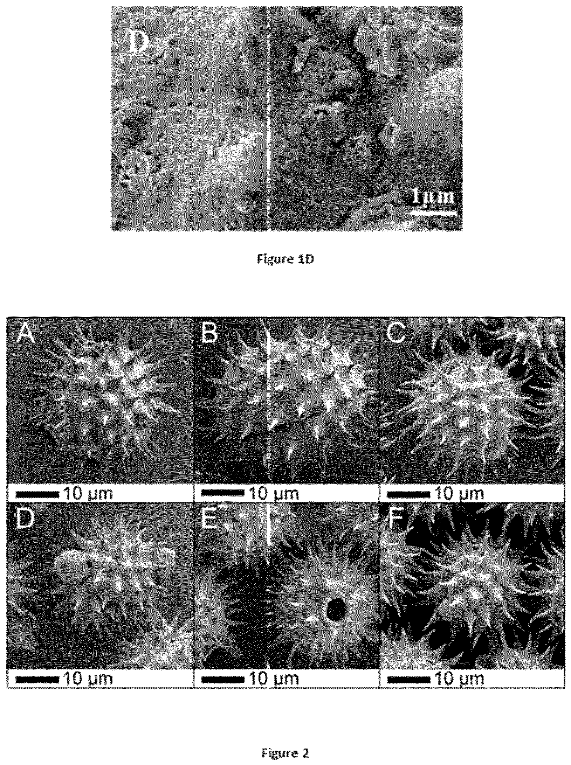

**Figures 1A, 1B, 1C****, and** **1D****:** Photographs showing the size and shape of the purified pollen disclosed herein on in different simulated biological media: A) before incubation; B) after incubation at 37°C in simulated intestinal fluid; C) after incubation at 37°C in simulated gastric fluid; and D) after incubation at 37°C in blood.
**Figure 2****:** Electron scanning micrograph of grains of sunflower (*Helianthus annuus*) pollen after different treatments. A: pollen washed with water. B: pollen after washing with cyclohexane (see Example 1). C: Pollen permeabilized with a hydrochloric acid solution between 40ºC and 80ºC for 5 minutes. D: Pollen permeabilized with a perchloric acid solution between 15ºC and 40ºC for 60 minutes. E: Pollen permeabilized with a phosphoric acid solution permeabilized by means of treatment with phosphoric acid above 60ºC for 120 minutes. F: Pollen washed with cyclohexane and subsequently treated with a proteinase K solution.

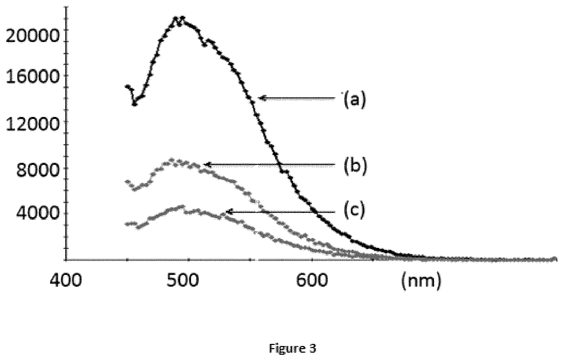

**Figure 3****:** Measurement of autofluorescence in pollen particles washed with water (a), and in capsules which have been subjected to an additional washing with chloroform/methanol (b), or with cyclohexane (c). See Example 1. The x-axis shows the wavelength in nm and the y-axis shows the units of absorption.

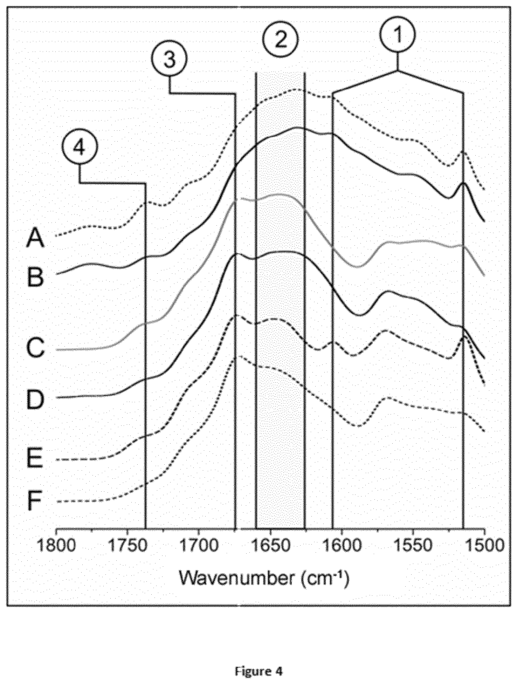

**Figure 4****:** Fourier transform infrared absorption spectra of pollen samples treated by heating with different acid solutions. **1.** Bands associated with sporopollenin 1606 and 1515 cm⁻¹. **2.** Amide I region where the protein C=O bond stretching bands are located. **3.** Band associated with alkene C=C stretching. **4.** Band characteristic of the C=O stretching vibration of lipids **A.** pollen washed with water. **B.** pollen after washing with cyclohexane (see Example 1) **C:** Pollen permeabilized with a phosphoric acid solution. **D:** Pollen permeabilized with a trifluoroacetic acid solution. **E:** Pollen permeabilized with a hydrochloric acid solution. **F:** Pollen permeabilized with a perchloric acid solution.

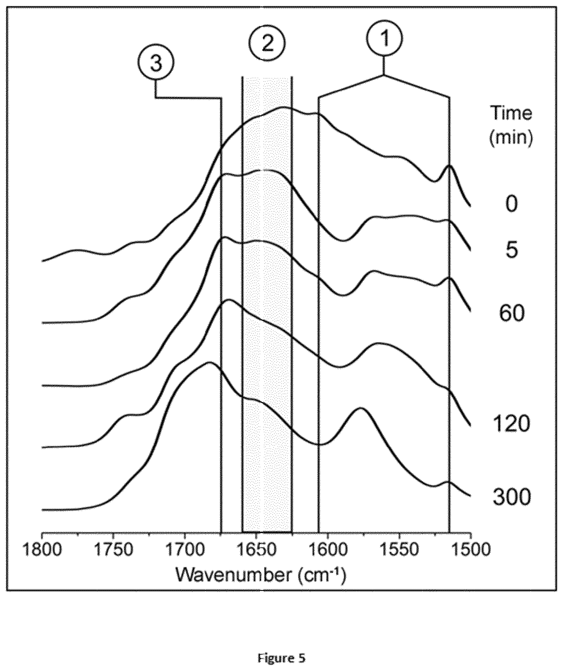

**Figure 5****:** Fourier transform infrared absorption spectra of pollen samples phosphoric acid solutions at different times. **1.** Bands associated with sporopollenin 1606 and 1515 cm⁻¹. **2.** Amide I region where protein C=O stretch bands are located. **3.** Band associated with alkene C=C stretching, 1670 cm⁻¹.

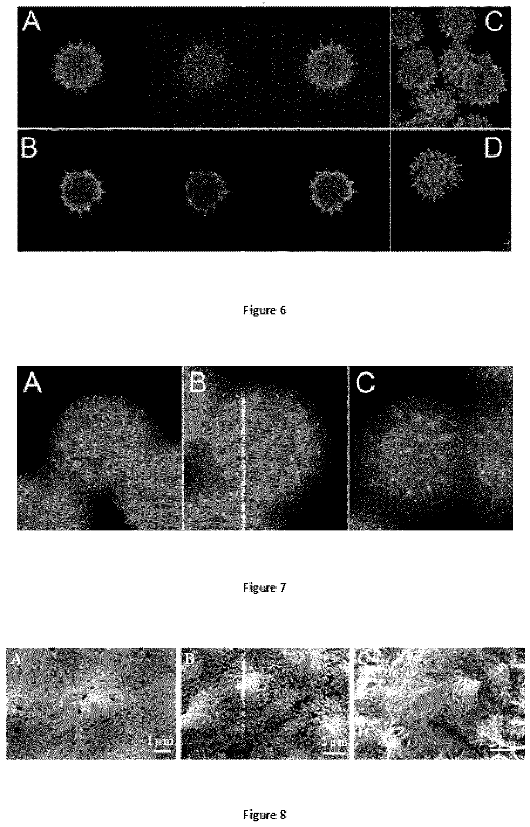

**Figure 6****:** Fluorescence micrograph of treated pollen samples. A. Internal section of a pollen grain treated with cyclohexane. B. Internal section of a pollen grain permeabilized with phosphoric acid. C. Pollen permeabilized with perchloric acid. D. External view of a pollen grain permeabilized with phosphoric acid.
**Figure 7****:** Fluorescence micrograph of pollen samples treated with enzymes. A. pollen permeabilized with acid solution. B. Pollen permeabilized and treatment with protease. C. Permeabilized pollen treated with carbohydrase and protease enzymes.
**Figure 8****:** SEM photographs of pollen particles associating A) chitosan nanoparticles; B) protamine nanocapsules; and C) protamine nanocapsules after lyophilization.

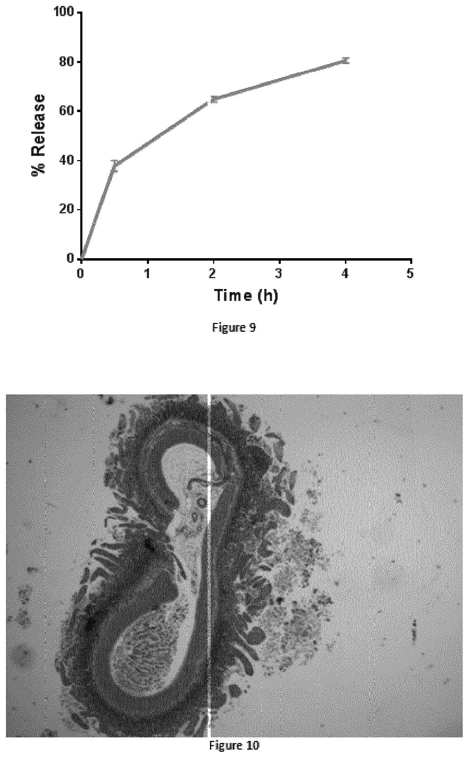

**Figure 9****:** Nanosystem release graph. Measurement of the fluorescence released in the supernatant after a centrifugation process at different times. The x-axis shows the times in hours and the y-axis shows the units of absorption.
**Figure 10****:** Microscopy photograph after hematoxylin/eosin staining in which it can be observed that the intestinal wall maintains its integrity after extraction and handling.

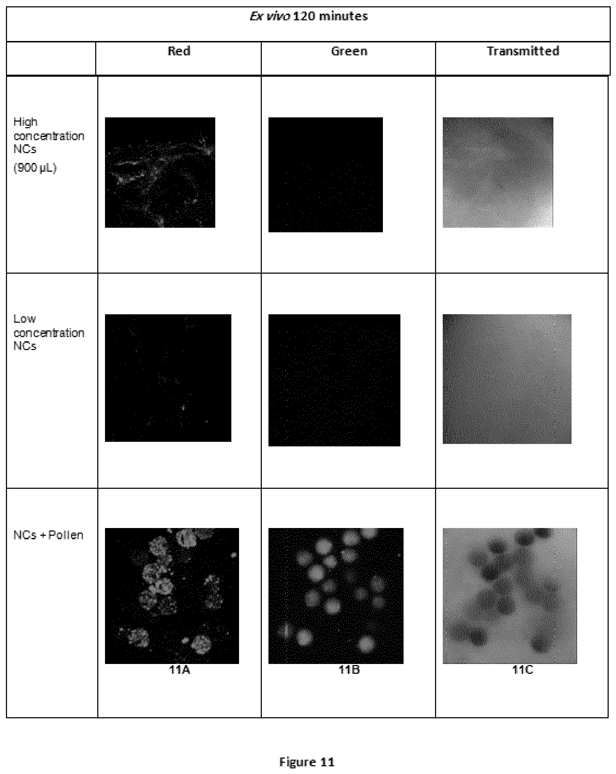

**Figure 11****:** *Ex vivo* confocal microscopy photographs taken at 120 minutes. The nanosystems are seen in the red channel (Figure 11A) and the pollen particles are seen in the green channel (Figure 11B). It can be seen how the nanosystems have been deposited on the purified pollen particles disclosed herein.

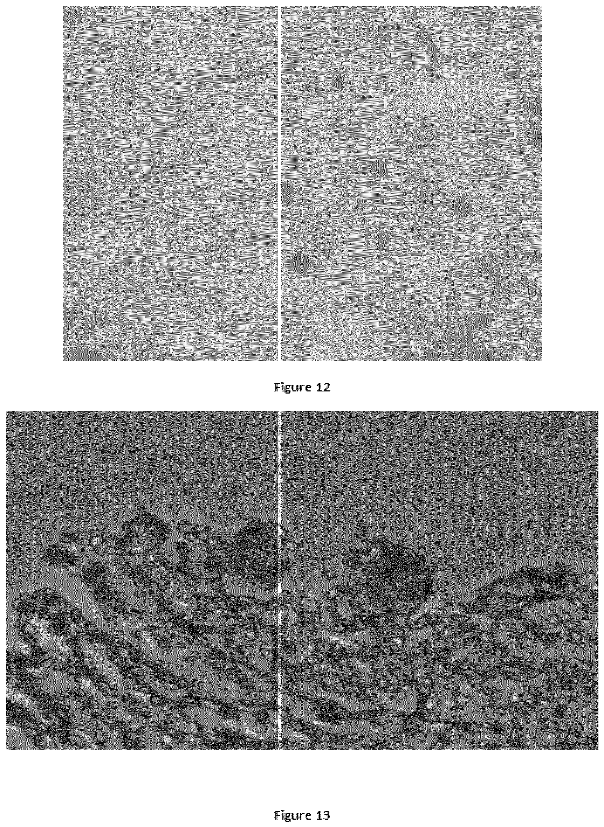

**Figure 12****:** Microscopy photograph in which it is observed that the pollen grains maintain their integrity and morphology *in vivo.*
**Figure 13****:** Microscopy photograph in which interaction between the pollen grains and the intestinal wall is observed.

### Detailed Description of the Invention

### Purified pollen particles

In the present disclosure, "purified pollen particle" is understood to be those pollen particles that have been treated to remove the lipid layer (*"pollenkitt"*) coating the outside thereof. Preferably, the treatment also completely or partially strips the cytoplasm from inside the particle. The treatment therefore preferably leaves the intine and exine substantially free of the cytoplasm and lipid layer. Said purification may not be complete and may still partially retain the lipid layer.

It is understood in the present invention that the purified pollen particles retain no more than 50%, preferably no more than 40%, for example, no more than 20%, no more than 10%, more preferably no more than 5% by weight of the lipid layer with respect to the original weight of the lipid layer. Preferably, at least 10 %, for example at least 20%, for example at least 30%, for example at least 40%, for example at least 50%, for example at least 60%, for example at least 70%, for example at least 80%, for example at least 90% by weight of the cytoplasm with respect to the original weight of the cytoplasm, has also been eliminated from the purified pollen particle.

The purified pollen particles comprise the intine layer and the exine layer, but do not comprise their lipid layer (*pollenkitt*) or preferably the cytoplasm either. The purified pollen particles are obtained through the simple pollen purification method that has been described above and comprises first washing the pollen particles with aqueous media and then with an organic solvent, without the pollen particles coming into contact with basic or acidic media at all (i.e., pH between 4.5 and 9).

Therefore, in the pollen particle purification method the water is at an essentially neutral pH, for example, at a pH between 5 and 9, preferably between 6 and 8, more preferably between 6.5 and 7.5.

Nor is it necessary for the purification process to involve washing at high temperatures. One having ordinary skill can adjust the conditions, and the water in the first step is preferably at a temperature comprised between 15°C and 60°C, preferably between 20°C and 45°C. Depending on the type of pollen, it is also possible to perform a second washing with water.

Various available organic solvents are at the disposal of one skilled in the art for performing the second washing. They can be cyclic or linear hydrocarbons such as, for example, those of formula CₙH₂ₙ₊₂ (non-cyclic, linear or branched) or CₙH₂ₙ (cyclic) having 5 to 20 carbon atoms (n=5-20). The examples which can be used in the present invention are pentane, hexane, heptane, octane, nonane, decane, cyclopentane, or cyclohexane, or mixtures thereof, for example, cyclohexane. The solvent used in the second step can also be an alcohol having, for example, 1 to 12 carbon atoms, for example, 1 to 4 carbon atoms. The examples that one having ordinary skill in the art can use in the present invention are methanol, ethanol, propanol, *tert*-butanol, or mixtures thereof. Halogenated hydrocarbons, i.e., hydrocarbons such as those defined at the beginning of this paragraph, but in which at least one of the hydrogen atoms has been substituted with a halogen (fluorine, chlorine, bromine, or iodine, preferably chlorine), are also non-limiting examples. The halogenated hydrocarbon used in the present invention can have the formula CₙH_{(2n+2-z)}X_{z} (non-cyclic, linear or branched) or CₙH_{2n-z}X_{z} (cyclic) where z is an integer (equal to or less than 2n+2 or 2n, as the case may be) and X is fluorine, chlorine, bromine, or iodine, preferably chlorine. Examples of solvents of this type are dichloromethane and chloroform. It is also possible to perform washing using mixtures of organic solvents in different proportions, for example, using a mixture of halogenated hydrocarbon:alcohol in proportions comprised between 1:20 and 20:1. An example can be the mixture of chloroform and methanol.

Other solvents which can be used in the present invention are those including a carbonyl or ester group and having a low molecular weight, for example, below 250 Da. Extended examples of these solvents are acetone or ethyl acetate.

Like in the case of washing with water, more than one washing with organic solvents can be performed. Washing with organic solvent is also preferably performed at low or moderate temperatures, for example, at a temperature comprised between 15°C and 60°C, preferably between 20°C and 45°C.

The method of the invention also thereby includes the possibility of repeating one or more times the washing with an aqueous medium of step (b) and/or the washing with an organic solvent of step (c) and/or step (d). For example, it may be suitable to perform more than one washing with an aqueous medium to facilitate the elimination of the cytoplasm. The method of the invention optionally includes one or more treatments with enzymes (for example, cellulases and/or amylases). These methods perforate the intine and thereby improve its porosity.

In that sense, a particular embodiment of the invention relates to a method further comprising an additional step c') comprising at least one of the following treatments: i) washing with an acid solution; ii) enzymatic treatment; iii) washing with a buffered aqueous medium at a pH comprised between 4.5 and 9; iv) washing with detergents. From these steps i)-iv), one skilled in the art may select one of them or perform combinations thereof according to their needs. One skilled in the art has at their disposal analysis and sufficient knowledge to enable performing said selection, as described in the examples. Like steps b) and c), these steps are intended for favoring pollen perforation for the purpose of eliminating the cytoplasm. As mentioned above, an additional advantage is that the methods of the present invention, including the washing of step c'), allow retaining the complete structure of the pollen wall, particularly the exine and the intine.

A preferred embodiment relates to a method further comprising an additional step c') comprising enzymatic treatment. The authors of the invention have found that enzymatic treatment allows greater control over pollen perforation.

A preferred embodiment therefore relates to a method further comprising an additional step c') which comprises washing with enzymes and washing with detergents.

A preferred embodiment relates to a method for preparing purified pollen particles, which method comprises the steps of
(b) washing a pollen particle in a buffered aqueous medium at a pH comprised between 4.5 and 9;
(c) washing with an organic solvent;
(c') enzymatic treatment,
for obtaining the purified pollen particle that retains the intine layer and the exine layer, but the lipid layer (*pollenkitt*) of which has been completely or partially eliminated.

The enzymatic treatments of the invention can be carried out with enzymes comprised in section 3 of the classification of EC numbers (Enzyme Commission numbers), preferably subsections 3.1, 3.2, 3.3, 3.4; and more preferably in subsection 3.2 with enzymes such as cellulases, and 3.4 with enzymes such as proteinases.

In a preferred embodiment, the enzyme is selected from hydrolases, carbohydrases, proteases cellulases, β-xylanases, β-glucanases, α-amylases, exo-1,4-α-glucosidases, lichenases, inulases, pectinases, polygalacturonases, and hemicellulases.

In a particular embodiment, the enzymes used in the processes described in the present invention are selected from bacterial subtilisins, alkaline proteases of bacillus, chymotrypsin, trypsin, proteinase K, protease OB, actinase E (pronase), protease S, pepsin, proteases from bovine pancreas, collagenase, elastase, thrombin, papain, bromelain, thermolysin.

As an additional advantage of enzymatic treatment, the authors have observed that the treatments of the previous steps are enhanced, very gentle, and furthermore more specific than the steps of washing with acidic solutions.

Therefore, the conditions under which the purified pollen particles disclosed herein can be prepared are fairly non-aggressive and readily implemented at the industrial level. Preferably, they do not comprise high pressures such that the process does not include any step in which the particles are subjected to pressures above 10 bars (10⁶ Pa). For example, all the steps of the process are performed at pressures below 5 bars (0.5 x 10⁶ Pa), preferably at pressures below 2 bars (0.2 x 10⁶ Pa), normally at atmospheric pressure (substantially 1 bar (10⁵ Pa)). This makes the process more appealing at the industrial level and prevents the pollen particles from breaking up. Preferably, the method for obtaining same does not involve any washing either in an aqueous medium the pH of which is below 4.5, preferably not below 5 or above 9, preferably above 8.

Given that the purified pollen particles disclosed herein can have applications in the pharmaceutical and food industry, the solvents are preferably pharmaceutically acceptable. This is not critical for obtaining the desired properties, and furthermore there is a need to take into account that these purified pollen particles will not be subjected to subsequent incubation and/or purification steps before being administered.

Each wash is performed according to the conventional techniques in this field and comprises forming a suspension of the pollen grains in water or in an organic solvent, usually followed by stirring. The suspension is usually incubated and centrifuged to then eliminate the supernatant. The resulting particles are usually dried, normally under moderate temperature conditions, for example, between 4°C and 60°C, for example, between 20°C and 40°C.

The purification method of the invention allows *a priori* incorporation of any nanosystem to the purified pollen particle. Said incorporation can occur as a result of the adsorption of the nanosystem on the surface of the purified pollen particle, or as a result of the absorption of the nanosystem into the purified pollen particle. Furthermore, the purified pollen particle can also incorporate a pharmaceutically acceptable active ingredient, a dietetically acceptable ingredient, or mixtures of both either as a component of the nanosystem itself or as an additional component. The purified particles disclosed herein can therefore incorporate the nanosystems, which may in turn comprise a pharmaceutically acceptable active ingredient, a dietetically acceptable ingredient, or mixtures of both. In a particular embodiment, the nanosystems can incorporate more than one active ingredient.

The incorporation of the nanosystem simply requires contacting it with the purified pollen particle obtained after steps (b) and (c). The method for incorporating nanosystems therefore comprise incubating the nanosystem in the presence of the purified pollen particle. Said incubation can be performed under dry conditions or in the presence of a liquid.

Therefore, the method of preparation can include the steps of
(b) washing a pollen particle in a buffered aqueous medium at a pH comprised between 4.5 and 9;
(c) washing with an organic solvent for obtaining the purified pollen particle that retains the intine layer and the exine layer, but the lipid layer (*pollenkitt*) of which has been completely or partially eliminated, preferably also including the complete or partial elimination of the cytoplasm; and(d) incubating the purified pollen particle in the presence of a nanosystem, for example, in the presence of a solvent.Alternatively, the method of preparation can include the steps of (b) washing a pollen particle in a buffered aqueous medium at a pH comprised between 4.5 and 9;(c) washing with an organic solvent for obtaining the purified pollen particle that retains the intine layer and the exine layer, but the lipid layer (*pollenkitt*) of which has been completely or partially eliminated; preferably also including the complete or partial elimination of the cytoplasm;(c") drying the purified pollen particle after steps (b) and (c); and impregnating the purified pollen particle with the nanosystems by optionally applying a vacuum.Additionally, the purified pollen particles thus obtained can be lyophilized. If necessary, a cryoprotectant, for example glucose or trehalose, can be added before proceeding with said lyophilization.

This purification method therefore allows the purified pollen particles to subsequently incorporate organic nanosystems which are capable of transporting the same to and through the mucosae, for example into the intestine, buccal, nasal, or ocular mucosa.

The pollen particles used as raw material to be purified can come from different species. Although the pollen particles have various sizes and morphologies, depending on the species of origin, the purification method of the present invention can be applied to any particle. Echinate pollen particles, which comprise needles with a length of at least one micrometer, for example, between 1 micrometer and 10 micrometers, or between 1 micrometer and 7 micrometers, generally between 1 micrometer and 5 micrometers, or between 1.5 micrometers and 2.5 micrometers, or between 1.5 micrometer and 2 micrometers, are preferred due to their morphology. For example, they can be particles from the family of *Helianthus,* for example, *Helianthus annuus* (sunflower).

Regarding size, availability, and morphology, the pollen particle can come from, for example, angiosperms or Magnoliophytas, i.e., flowering plants. Among angiosperms or Magnoliophytas, the pollen particles can belong to monocotyledon, Chloranthaceae, Ceratophyllaceae, Magnoliidae, or eudicotyledon classes, particularly eudicotyledon, with spinose species being preferred. The most suitable eudicotyledon families are Annonaceae, Malvaceae, Meliaceae, Tamaricaceae, Asteraceae, Oleaceae, or Caprifoliaceae.

For example, spinose species that are particularly useful for the purposes of the invention are one or more selected from the group consisting of
- Magnoliophyta eudicotyledons Asterales Asteraceae Ambrosia sp.
- Magnoliophyta eudicotyledons Asterales Asteraceae Helianthus sp.
- Magnoliophyta eudicotyledons Asterales Asteraceae Pectis sp.
- Magnoliophyta eudicotyledons Asterales Asteraceae Tagetes sp.
- Magnoliophyta eudicotyledons Asterales Asteraceae Stevia sp.
- Magnoliophyta eudicotyledons Asterales Asteraceae Calendula sp.
- Magnoliophyta eudicotyledons Asterales Asteraceae Parthenium sp.
- Magnoliophyta eudicotyledons Asterales Asteraceae Balsamorhiza sp.
- Magnoliophyta eudicotyledons Asterales Asteraceae Cirsium sp.
- Magnoliophyta eudicotyledons Asterales Asteraceae Balsamorhiza sp.
- Magnoliophyta eudicotyledons Asterales Asteraceae Arnica sp.
- Magnoliophyta eudicotyledons Asterales Asteraceae Ambrosia sp
- Magnoliophyta eudicotyledons Asterales Asteraceae Aster sp.
- Magnoliophyta eudicotyledons Asterales Asteraceae Bidens sp.
- Magnoliophyta eudicotyledons Asterales Asteraceae Clibadium sp.
- Magnoliophyta eudicotyledons Asterales Asteraceae Cosmos sp.
- Magnoliophyta eudicotyledons Asterales Asteraceae Doronicum sp.
- Magnoliophyta eudicotyledons Asterales Asteraceae Echinacea sp.
- Magnoliophyta eudicotyledons Asterales Asteraceae Erechtites sp.
- Magnoliophyta eudicotyledons Asterales Asteraceae Erigeron sp.
- Magnoliophyta eudicotyledons Asterales Asteraceae Gaillardia sp.
- Magnoliophyta eudicotyledons Asterales Asteraceae Inula sp.
- Magnoliophyta eudicotyledons Asterales Asteraceae Leucanthemopsis sp.
- Magnoliophyta eudicotyledons Asterales Asteraceae Liatris sp.
- Magnoliophyta eudicotyledons Asterales Asteraceae Pulicaria sp.
- Magnoliophyta eudicotyledons Asterales Asteraceae Scorzonera sp.
- Magnoliophyta eudicotyledons Asterales Asteraceae Tetragonotheca sp.
- Magnoliophyta eudicotyledons Alismatales Araceae Ulearum sp.
- Magnoliophyta eudicotyledons Alismatales Araceae Zomicarpa
- Magnoliophyta eudicotyledons Alismatales Araceae Pinellia sp.
- Magnoliophyta eudicotyledons Magnoliales Annonaceae Annonaceae sp.
- Magnoliophyta eudicotyledons Sapindales Meliaceae Melia sp.
- Magnoliophyta eudicotyledons Caryophyllales Tamaricaceae Tamarix sp.
- Magnoliophyta eudicotyledons Lamiales Oleaceae Olea sp.
- Magnoliophyta eudicotyledons Dipsacales Caprifoliaceae Lonicera sp.
- Magnoliophyta eudicotyledons Malvales Malvaceae Malope sp.
- Magnoliophyta eudicotyledons Malvales Malvaceae Abutilon sp.
- Magnoliophyta eudicotyledons Malvales Malvaceae Hibiscus sp.
- Magnoliophyta eudicotyledons Malvales Malvaceae Lavatera sp.
- Magnoliophyta eudicotyledons Malvales Malvaceae Sphaeralcea sp.
- Magnoliophyta eudicotyledons Malvales Malvaceae Malva sp.
- and mixtures thereof.

The pollen particles used as raw material in the invention can have different sizes, for example, a mean diameter comprised between 1 micrometer and 400 micrometers, 1 micrometer and 300 micrometers, or between 1 micrometer and 200 micrometers, or between 1 micrometer and 100 micrometers, for example, between 10 micrometer and 50 micrometers, for example, between 15 micrometers and 40 micrometers, or between 20 micrometers and 30 micrometers, or between 25 and 30 micrometers.

The purified pollen particles, the nanosystems, the pharmaceutically acceptable active ingredient, or the dietetically acceptable ingredient, can be associated with a marker, for example, a fluorescent marker. Non-limiting examples of fluorescent markers of this type are amine-reactive dyes (for example, Alexa Fluor^{®}), TAMRA, or Cy-dyes. This allows tracking each of these markers together or separately.

### Nanosystems

Purified pollen particles disclosed herein have the capacity to incorporate nanosystems, preferably organic nanosystems. In the present disclosure, the term "nanosystem" as it is used herein is a colloid, i.e., a lyophobic colloidal particle with a mean particle diameter between 1 nm and 500 nm, more preferably between 40 nm and 400 nm, wherein said mean diameter is measured by means of photon correlation spectroscopy in a Malvern Instruments Nanosizer, and being lyophobic, i.e., not dissolved in its external phase (Paul Hiemenz, Raj Rajagopalan, Principles of colloid and Surface chemistry, 3rd Ed., Marcel Dekker, Inc., New York, 1997). In the context of this invention, the colloidal system is intended for the composition thereof to have at least one fluorescent marker or a pharmacologically active molecule. In the present invention, said term is considered to include, for example, "nanocapsule", "nanoparticle", "vesicle", "micelle", "nanoemulsion", "liposome," or "ultrafine particle". One having average skill in the art also understands that organic nanosystems are those nanosystems the components of which are at least organic in part, i.e., based on molecules comprising carbon and hydrogen, for example, proteins, carbohydrates, or lipids. See for example, Kumar R, Lal S J Nanomater Mol Nonotechnol 2014, 3, 4. The nanosystems disclosed herein can be for example polymeric nanosystems. Polymeric nanosystems are widely described in the literature and are known to one skilled in the art, as described for example by Pinto Reis et al., in Nanomedicine: Nanotechnology, Biology, and Medicine 2(2006) 8-21. Pinto Reis, *et al.* 2006.

The polymeric nanosystem mean diameter polydispersity index which can be used in the present invention is comprised between 0.1 and 0.5, wherein the polydispersity index is measured by means of the technique called photon correlation spectroscopy measured in a Malvern Instruments Nanosizer. For the same reasons, the nanosystem is preferably substantially biodegradable and presents low or zero toxicity. It is therefore considered that the nanosystem is biodegradable when at least one of its components complies with commonly accepted standards, for example, the biodegradability standards drafted by different standardization entities (ISO, CEN, ASTM, DIN, etc.), for example, it is 90% biodegradable after 6 months according to UNE-EN-ISO standard 14852:2005 (determination of the ultimate aerobic biodegradability of plastic materials in an aqueous medium), i.e., 90% of the carbon (C) atoms present in the component were converted to carbon dioxide after six months under the conditions defined in the standard.

One skilled in the art will recognize that there are various classes of nanosystems that can be used in the present invention, many of which are commercially available or can be prepared by methods described in the state of the art. They may be of a matrix type, i.e., ionically crosslinked polymer networks, for example, containing water-soluble polymers. Non-limiting examples of nanosystems of this type are those based on polysaccharides, for example, chitosan (chitosan nanoparticles) or on polyamino acids, for example, protamine (protamine nanocapsules). See, for example, the method described in Calvo P, Remuñán-López C, Vila-Jato JL, lonso MJ J Appl Polym Sci 1997, 63, 125-132 or in Thwala L **(2016)** *Protamine nanocapsules as carriers for oral peptide delivery.*

Nanosystems comprising a liquid core surrounded by a coating layer can also be used in the present invention. The core can incorporate different oils, lipid material (for example, fatty acids or phospholipids, or mono-, di- or triglycerides), in combination with non-ionic surfactants. The coating layer can be a polymer, for example, protamine. See, for example, the methods described in patent application PCT/ES2013/070885.

Other nanosystems suitable for use in the present invention are solid nanoparticles, such as for example, chitosan nanoparticles, particularly those described in documents ES2481940B1, ES2093562, Csaba et al, Journal of Controlled Release, 2017, 245, 62-69; Marcos García-Fuentes, M.J.A, Journal of Controlled Release, 2012, 161 (2), 496-504. One skilled in the art will know how to prepare these and other polymeric nanosystems according to Nanomedicine: Nanotechnology, Biology, and Medicine 2(2006) 8-21. Pinto Reis, et al. 2006.

The nanosystems are preferably pharmaceutically acceptable for the applications proposed in the present invention.

### Applications

Researchers have found that the purified pollen particles disclosed herein are stable *in vivo* and have a surprising intestinal mucosa penetration capacity, rendering them an excellent transmucosal administration platform for nanosystems, especially for oral administration. The present invention therefore includes the use of the delivery systems of the invention, for manufacturing a medicinal product, that is, a delivery system of the inventionfor use as a medicinal product. The delivery systems of the invention are therefore particularly suitable for use in manufacturing a medicinal product for transmucosal administration, such as oral administration, ocular administration, or nasal administration.

For example, the delivery system of the invention can be used for ocular administration, including the use thereof for manufacturing a medicinal product for the treatment of ocular diseases, for example, for curing ocular wounds or macular disease.

The delivery system of the present invention can also be used for the nasal administration of antigens, which is particularly of interest for the administration of vaccines, which would relieve the healthcare burden involved with parenteral administration, particularly in developing countries.

Given its good absorption and penetration in the intestinal mucosa, a particularly suitable use for the delivery system of the invention is the manufacture of a medicinal product for oral administration and/or the manufacture of a medicinal product for the controlled release of an active ingredient, preferably for its delivery in the digestive tract. The term "digestive tract" is understood in the present invention to mean the system through which any orally administered active ingredient traverses from the time it is taken until it is excreted. It therefore includes the mouth, throat, esophagus, stomach, small intestine, and large intestine, among others. For example, the delivery system of the invention can be useful for the administration of molecules for which only parenteral administration means are known, for example for the administration of antidiabetic peptides. The incorporation of these active ingredients in nanosystems and the incorporation of the latter in turn in the purified pollen particles, allows transport to the epithelium and the controlled release thereof.

One situation in which the transmucosal administration can offer unique advantages is the in the administration of antigens. Mucosal surfaces such as the respiratory or gastrointestinal tract represent the main entry pathway for many pathogens and play an integral role in the development of effective defense mechanisms against same. In fact, a combined immune response which entails systemic and mucosal immunity is best achieved by means of the transmucosal administration of antigens.

The term "active ingredient" refers to a substance used for treating, curing, or preventing a medical condition or a disease. It also covers in the context of the present invention those substances which are used in diagnostic tests. Said active ingredient is part of the nanosystem, and can be, for example, a contrast agent or a vaccine. In one embodiment of the invention, the active ingredient is a high molecular weight compound, for example, a protein, a peptide, a lipid, an antibody, or a nucleic acid.

Therefore, the present invention also relates to a composition comprising the delivery system of the invention and a pharmaceutically acceptable excipient. In another aspect, the invention relates to a composition comprising the delivery system of the invention and a dietetically acceptable excipient.

The term "excipient" refers to a diluent or adjuvant with which the active ingredient is administered. Such pharmaceutical excipients can be sterile liquids, such as water or oils, including those of a petroleum, animal, plant, or synthetic origin, such as peanut oil, soybean oil, mineral oil, sesame seed oil, and the like. Suitable pharmaceutical excipients are described in "Remington's Pharmaceutical Sciences" by E.W. Martin. Said excipients can also be considered the ancillary substances needed to manufacture the desired dosage form. The nature and the amounts thereof depend on, among other factors, the chosen pharmaceutical dosage form. Said pharmaceutical dosage forms of the pharmaceutical composition will be manufactured according to conventional methods known to one skilled in the art. A review of different active ingredient administration methods, excipients to be used, and methods for producing them can be found in "Tratado de Farmacia Galénica", C. Fauli i Trillo, Luzán 5, S.A. de Ediciones, 1993.

The term "pharmaceutically acceptable" refers to molecular entities and compositions which are physiologically tolerable and do not normally cause an unwanted allergic reaction or the like, such as stomach discomfort, dizziness, and the like, when administered to a human being. Preferably, as it is used herein, the term "pharmaceutically acceptable" means approved by a regulatory agency of the federal or state government or listed in the US Pharmacopoeia or other generally recognized pharmacopoeia for use in animals and more particularly in human beings.

Similarly, the term "dietetically acceptable" refers to molecular entities and compositions which are physiologically tolerable, preferably approved for human consumption by a regulatory agency for use for nutritional purposes in animals and more particularly in human beings. That is, use of delivery system of the present invention for the administration of a dietetically acceptable ingredient excludes therapeutic uses and can serve, for example, for the administration of a flavor masking agent.

The present disclosure therefore includes methods for the treatment of a subject in need of treatment by means of the administration of a therapeutically effective amount of delivery system of the invention. The term "treatment" or "treating" in the context of the present invention refers to the administration of the pharmaceutical compositions of the invention for preventing, reducing, or eliminating one or more symptoms associated with a medical condition or a disease. The term "treating" also covers the elimination, reduction, or prevention of sequelae of said disease or medical condition. The term "reducing" is understood in the context of the present invention to mean the improvement in the situation of the patient, evaluated either by subjective means (perception of the patient with regard to a particular aspect or with regard to their general condition) or by objective means, for example, biological parameters, for example, levels of an analyte in specific fluids.

The term "therapeutically effective amount" refers to the amount of active ingredient calculated for producing the desired effect and will generally be determined, among others, by the actual characteristics of the active ingredient used and the therapeutic effect to be obtained. In a particular embodiment, the active ingredient dosage administered to a subject in need of treatment is in the range of 10⁻¹⁰ to 10¹⁰ mg/kg of body weight, usually between 10⁻³ and 10³ mg/kg of body weight.

The medicinal product comprising the delivery system of the invention can be found in any suitable form for administration to humans and/or animals, preferably humans, including babies, children, and adults, and it can be prepared by standard methods known to those skilled in the art, for example, "Pharmaceutics: The Science of Dosage Forms, second edition, Aulton, M.E. (ed.) Churchill Livingstone, Edinburgh (2002); "Encyclopedia of Pharmaceutical Technology", second edition, Swarbrick, J. and Boylan J.C. (eds.), Marcel Dekker, Inc. New York (2002); "Modern Pharmaceutics", fourth edition, Banker G.S. and Rhodes C.T. (eds.) Marcel Dekker, Inc. New York 2002 and "The Theory and Practice of Industrial Pharmacy", Lachman L., Lieberman H. and Kanig J. (eds.), Lea & Febiger, Philadelphia (1986). The composition of the medicinal product may vary depending on the route of administration.

The pharmaceutical composition of the invention can be administered in a plurality of pharmaceutical dosage forms, for example, solid, liquid dosage forms, etc. Non-limiting illustrative examples of said pharmaceutical dosage forms of the pharmaceutical composition of the invention include oral drops (suspension, emulsion, etc.); oral formulations (liquid formulations, suspension, emulsion, gel, paste, powder, etc.); oral lyophilisate; oral gum; powder for oral suspension; granules; gastro-resistant granules; prolonged release granules; modified release granules; granules for oral suspension; powder and solvent for oral solution or suspension; syrup; powder for syrup; granules for syrup; tablets (for example, soluble tablet, dispersible tablet, coated tablet, film-coated tablet, effervescent tablet, orodispersible tablet, gastro-resistant tablet, prolonged release tablet, modified release tablet, oral tablet, chewable tablet, etc.); effervescent powder or granules; sachet, capsule; pills; intraruminal extended release device; intraruminal pulsatile release device; sucking block; premix for medicated feeding staff; dragées; suspension, drops, aerosol, gel, paste, oromucosal capsule, etc.; aerosol, sublingual tablet, etc.; mouthwash; gel, gingival paste, etc.; lozenge; pill; dental gel, cotton swab, insert, powder, suspension, emulsion, etc.; toothpaste; cream; gel; ointment; ocular cream; ocular gel; ocular ointment; eye drops (powder and solvent for suspension, lotion, solvent for reconstituting a lotion, etc.); ophthalmic insert; ear cream; ear gel; ear ointment; ear drops (suspension, emulsion, powder, etc.); ear aerosol (suspension, etc.); ear irrigation (emulsion, etc.); ear buffer; ear cotton swab; nasal cream; nasal gel; nasal ointment; nasal drops (suspension, emulsion, etc.); nasal powder; nasal aerosol (suspension, emulsion, etc.); nasal irrigation, nasal cotton swab; vaginal cream; vaginal gel; vaginal ointment; vaginal foam; vaginal suspension; vaginal emulsion; hard or soft vaginal capsule; vaginal tablet; effervescent vaginal tablet; vaginal administration system; rectal cream; rectal gel; rectal ointment; rectal foam; rectal suspension; rectal emulsion; powder for rectal solution; powder for rectal suspension; tablet for rectal suspension; suppository; rectal capsule; nebulizer suspension; powder for nebulizer suspension; nebulizer emulsion; pressurized inhalation (suspension, emulsion, etc.); powder for inhalation; powder for inhalation (hard capsule); predispensed powder for inhalation; gel for injection; suspension for injection; emulsion for injection; powder for suspension for injection; powder and solvent for suspension for injection; solution for infusion; emulsion for infusion; implantation tablet; bladder irrigation; powder for bladder irrigation; urethral gel; urethral cotton swab; endotracheopulmonary instillation (solution); endotracheopulmonary instillation; endotracheopulmonary instillation (suspension); endocervical gel; powder and solvent for endocervical gel; intramammary suspension; intramammary emulsion; intramammary ointment; cotton swab for nipples; intrauterine administration system; etc.

### Examples

Specific examples of pollen purification (Example 1), of the preparation of nanosystems suitable for the present invention (Example 2), and their incorporation in purified pollen particles to form vehicles for administration (Example 3) are described below. The results obtained in an *ex vivo* example (Example 4) designed to evaluate the purified pollen particles disclosed herein, as well as *in vitro* and *in vivo* experiments showing the stability of the purified pollen particles disclosed herein (Examples 5 and 6, respectively), are also shown. These examples serve to illustrate embodiments of the invention, but in no case should they be considered as limiting.

### Example 1: Purification of purified pollen particles

### Example 1.1. Purification: aqueous and organic washing

Pollen particles of sunflower, *Helianthus annuus,* were used as the basis for the purification of pollen particles.

### Step 1

Ten (10) mg of pollen particles were suspended in 20 ml of milli-Q water previously heated at 37°C for 30 seconds under stirring. The suspension was kept under horizontal stirring throughout the preceding night at room temperature and was centrifuged the next day at 2500 rpm at 15°C for 10 minutes (Hettich centrifuge with rotor 1689). The supernatant was discarded and the process was repeated, washing with 10 ml of milli-Q water at 37°C. The suspension was centrifuged again at 2500 rpm, at 15°C for 5 minutes. The supernatant was discarded and the resulting particles were dried at 37°C for one night.

### Step 2

The material of Step 1 was then re-suspended in milli-Q water to prevent the formation of aggregates and washed with organic solvent. Two examples are presented in this case, the first example using cyclohexane and the second example using a chloroform/methanol mixture.

In the first case 800 microliters of cyclohexane were added to 75 mg of the material from Step 1. The resulting suspension was stirred for 30 seconds, centrifuged at 14000 rpm at 15°C for 1 minute (Hettich centrifuge with rotor 1689), to later discard the supernatant. The resulting particles were dried at 37°C for one night.

In the second case, 10 ml of a chloroform/methanol mixture (3:1; v/v) were added to 50 mg of the material from Step 1. The suspension was stirred for 30 seconds and filtered by means of a membrane with pore size of 0.22 micra, which was washed with 5 ml of the chloroform/methanol mixture. After 120 minutes of vacuum drying, the material was recovered from the filter.

### Characterization

The stability of the material thus obtained was evaluated with respect to *in vivo* conditions. Specifically, it was incubated for 2 hours at 37°C in the following media: simulated intestinal fluid at pH 6.8, simulated gastric fluid at pH 1.2, postprandial blood and under the conditions of the upper small intestine, i.e., simulation of a feeding situation in the intestinal fluid (pH 5.8). After various tests, the material was evaluated by SEM, without observing significant variations, thus confirming that the purified pollen particles prepared by the simple purification method of the invention are stable under *in vivo* conditions in media as aggressive as gastric and intestinal media. The formation of precipitates of salt and other products on the surface of the purified pollen particles was observed only in the case of incubation in gastric fluid. The photographs of the particles obtained after incubation in simulated intestinal fluid at pH 6.8, simulated gastric fluid at pH 1.2, and blood are shown in Figures 1B, 1C, and 1D, respectively, and can be compared with the photograph obtained before incubation (Figure 1A).

To evaluate the results of this method, the morphology of the material obtained from Step 1, washed only with water, was compared with the resulting material from Step 2, according to the invention, washed with water and organic solvent. SEM photographs showed poorly defined pores in the first case, showing that the layer coating the exine is not eliminated completely with water alone, and an additional wash with an organic solvent, an acidic solution, an enzymatic treatment, or a detergent solution is necessary. See Figure 2.

For this comparison, the autofluorescence of the material obtained from Step 1, washed only with water, and the autofluorescence of the resulting material from Step 2, according to the invention, washed in an aqueous medium at an approximately neutral pH and organic solvent were also measured. In all cases, the emission of fluorescence occurs at around 500 nm, but in the case of the material prepared by the method of the invention, the level of autofluorescence was significantly lower, about 8000 units in the case of the additional wash with the chloroform/methanol mixture, and about 4000 units in the case of the additional wash with cyclohexane. See Figure 3. This shows the different modification that is done to the system.

### Example 1.2. Washing with acidic aqueous medium

### Example 1.2.1 permeabilization of pollen using acidic solutions above 40ºC and below 80ºC

Seventy (70) mg of purified pollen were suspended in 1 ml of an acidic solution (for example, phosphoric acid, sulfuric acid, perchloric acid, or hydrochloric acid) and heated at 70°C for 5 minutes under stirring. Depending on the acid and the desired degree of permeabilization, the process may take up to 120 minutes.

The pollen was centrifuged at 14000 rpm for 10 minutes, the supernatant was eliminated and suspended in water for additional washing. The pollen sample was homogenized with the help of a vortex mixer and centrifuged again under the same conditions. The washing process in which 1 ml of water was added to the sample was repeated several times. The washing process and the process of removing acid residues can be performed by dialysis against water. The resulting particles were dried at 37°C overnight.

Alternatively, the permeated pollen could be recovered by filtration using filters compatible with the selected acid. The permeated pollen was suspended in water and/or buffer and filtered again in a washing step. Additionally, the permeated pollen can be completely neutralized using a buffer for washing with a pH comprised between 3 and 11, preferably between 4.5 and 9. After neutralization, the pollen was washed again with water. The material was recovered from the filter and dried.

### Example 1.2.2: permeabilization of pollen using solutions at temperature between 15 and 40ºC

Seventy (70) mg of purified pollen were suspended in 1 ml of concentrated acid (for example, phosphoric acid, perchloric acid, or hydrochloric acid) and heated at 37°C for 1 hour under stirring. The process can alternatively be performed at room temperature.

### Characterization

Permeabilization of the sunflower pollen grains and their morphology was evaluated by means of optical, fluorescence, confocal, and scanning electron microscopy (SEM) techniques. The protein content of the samples was evaluated by elemental analysis and Fourier-transform infrared spectrometry (FTIR). See Figures 4, 5, and 6.

Through optical microscopy and the autofluorescence of the different layers of pollen (exine: blue; intine: red), it could be observed that evagination of the intine layer in the germination pore of the pollen occurred after treatment with acidic solutions, forming rounded protuberances. More prolonged treatments produce an apical aperture, eventually completely exposing the colporate aperture of the pollen. It was observed that said treatments completely or partially strip the cytoplasm from inside the pollen. See Figure 7.

The IR spectrum of untreated pollen and pollen after being treated with cyclohexane are similar; however, it is observed that the band at 1735 cm⁻¹ disappears, which demonstrates lipid extraction, given that this band is described as a characteristic of the C=O stretching vibration of lipids, triglycerides, and alkyl esters. After treatment with the acidic solution, a decrease in the band intensity at 1630 cm⁻¹ in the amide I region, characteristic of proteins, is observed. Likewise, in prolonged treatments in acidic solution a decrease in bands at 1606 and 1515 cm⁻¹, characteristic of sporopollenin, is observed, which suggests that the exine starts to degrade, as can be observed in the scanning electron microscopy micrographs of the pollen treated for 300 minutes. See Figures 4 and 5.

These results demonstrate that it is possible to control the degree of aperture and stripping of the pollen grains by changing the conditions of a treatment with an acidic solution. These permeated particles must show a nanosystem loading capacity greater than those that are untreated given that the useful load space is enlarged.

### Example 1.3. Enzymatic washing

For the purpose of eliminating protein residues (allergens) and increasing the pore size obtained in Example 1.2.1, two non-limiting examples of enzymatic treatment of previously pre-treated pollen particles of sunflower (*Helianthus annuus*) either permeabilized (such as those obtained in Example 1.2) or alternatively purified from lipids (such as those obtained in Example 1.1) are provided below.

Non-limiting examples of proteases are proteinase K, trypsin, papain, bromelain, alkaline protease. The enzymatic treatments can be carried out with the enzymes comprised in section 3 of the classification of EC (Enzyme Commission) numbers, preferably subsections 3.1, 3.2, 3.3, 3.4, and more preferably in subsection 3.2 with enzymes such as cellulases and 3.4 with enzymes such as proteinases.

Seventy (70) mg of pollen pretreated either with an acidic solution (product of Example 1.2) or, alternatively, with organic solvents (product of Example 1.1) were suspended in 1 ml of buffer at a pH suitable for the hydrolase used, at acid pH for carbohydrase enzymes or at alkaline pH in the case of protease. The samples were incubated in an orbital agitator at 1000 rpm for a period comprised between 10 minutes and 120 minutes, at the optimal temperature described for each hydrolase, generally 55ºC for carbohydrase enzymes and 37ºC for proteases.

The enzymatically treated pollen was purified as described in step 2 of Example 1.2.1.

### Step 2

In the event of a combined treatment of carbohydrase and protease enzymes, the pollen particles obtained after the treatment of step 1 can be subjected to subsequent treatment with proteases. To that end, the purified product from step 1 is resuspended in 1 ml of buffer at a pH between 7 and 9, preferably 8, at a concentration of 10 mM to 1 M. The reactions were incubated in an orbital agitator at 1000 rpm for a period comprised between 10 minutes and 120 minutes, at the optimal temperature of the protease.

### Characterization

After treatment with hydrolases, it could be observed that the prior treatment was improved Pore formation in the protuberances observed in the permeabilized pollen was achieved with gentler treatments (shorter time and/or lower temperature). Through kinetic concentration and reaction time studies, the increase in activity could be observed, reflected as morphological changes in the pretreated pollen grains. Given the substrate specificity of enzymes, treatments with carbohydrases and/or proteases allow modulating the effect of hydrolysis in a selective and more controlled manner than with the acid hydrolysis that occurs with other treatments given that the latter simultaneously affects polysaccharides (exine and intine) and proteins (cellular components, protein allergens, etc.).

### Example 1.4. Washing with detergents

For the purpose of eliminating protein residues (allergens), two non-limiting examples of enzymatic treatment of previously pretreated pollen particles of sunflower (*Helianthus annuus*) either purified from lipids (such as those obtained in Example 1.1), permeabilized (such as those obtained in Example 1.2), enzymatically treated (such as those obtained in Example 1.3), or their combined use of the treatments described above, for example, co-incubation with detergent and proteases, are provided below. Non-limiting examples of the detergents include the ionic type such as sodium dodecyl sulfate (SDS), deoxycholate, cholate, sarcosyl; the non-ionic type such as Triton X-100, n-dodecyl-β-D-maltoside (DDM), digitonin, Tween-20; of the zwitterionic type such as 3-[(3-cholamidopropyl)dimethylammonio]-1-propanesulfonate (CHAPS); and the chaotropic type, such as urea, among others.

### Step 1

Seventy (70) mg of pretreated pollen particles were suspended in 1 ml of buffer with different detergents allowing protein elimination, such as detergents of the ionic type such as sodium dodecyl sulfate (SDS), deoxycholate, cholate, sarcosyl; of the non-ionic type such as Triton X-100, DDM, digitonin, Tween-20; of the zwitterionic type such as CHAPS; and of the chaotropic type such as urea, among others. The suspension was kept under stirring at 37ºC for 120 minutes under stirring and centrifuged at 14000 rpm at 20°C for 10 minutes. The supernatant was discarded and the process was repeated, washing with 10 ml of milli-Q water at 37°C. The suspension was centrifuged again at 14000 rpm, at 15°C for 10 minutes. The supernatant was discarded and the resulting pollen particles were dried at 37°C for one night.

### Step 2

Alternatively, 70 mg of pollen pretreated particles were incubated in 1 ml of buffer with detergents compatible with proteolytic activity, such as Urea, Tween-20, Triton X-100, and SDS. As an example of the protease, proteinase K was used at a concentration of 1 mg/ml. The reaction was incubated for a time comprised between 5 and 120 min at 37°C under stirring. The sample was then purified as explained in Step 1.

### Example 2: Examples of organic nanosystems that can be encapsulated

Two non-limiting examples of the preparation of organic nanosystems that can be encapsulated are provided below.

### Example 2.1: Formulation of chitosan/sodium triphosphate (CS/TPP) nanoparticles

The chitosan/sodium triphosphate nanoparticles were obtained using a CS/TPP ratio (5:1) (w/w), by means of the ionic gelation technique, according to patent application WO9804244. A solution of 2 mg/ml of CS and 1.2 mg/ml of TPP in milli-Q water was prepared. To obtain the nanoparticles, 0.5 ml of TPP were poured over 1.5 ml of CS under magnetic stirring and were left under stirring for 10 minutes at 25ºC. The nanoparticles are formed spontaneously.

### Example 2.2: Protamine nanocapsules

Protamine nanocapsules were prepared by means of the solvent diffusion technique according to patent ES2481940 in accordance with the following steps:
1. An aqueous phase (10 ml) of protamine was prepared with a final concentration of 1.5 mg/ml. This solution was kept under magnetic stirring at room temperature.
2. An organic phase of 3 ml of PEG-stearate 40 (5.333 mg/ml) and 20 uL sodium glycocholate (250 mg/ml) was prepared and mixed with a vortex (5 seconds), and then between 20 and 100 µl of Miglyol^{®} oil were added, depending on the desired size. The mixture was stirred again by vortex and finally 1.98 ml of acetone are added.

Optionally, for preparing fluorescent nanocapsules, 20 microliters of the marker 1,1'-dioctadecyl-3,3,3',3'-tetramethylindocarbocyanine perchlorate (DiD; 2.5 mg/ml) are added to the organic phase in this step.

3. The organic phase was rapidly transferred to the previously prepared aqueous phase by means of continuous injection or simple addition depending on the desired size. The nanocapsules are formed spontaneously.

4. After maintaining the suspension for 10 minutes, the organic solvents were eliminated by rotoevaporation. After this step, isolation can optionally be performed (for example, by ultracentrifugation: 30,000 rpm, 1 hour, 15ºC)

### Characterization

The properties of the nanoparticles and nanocapsules are summarized in Table 1 below.

**Table 1**

| **Example** | **Size (nm)** | **Polydispersity index - PdI** | **Zeta potential (mV)** | **Fluorescent agent DiD (EE %)** |
|---|---|---|---|---|
| **Example 2.1** | 187 | 0.3 | 0 | - |
| **Example 2.2** | 101 ± 3 | 0.05 | +3 ± 1 | >60 |
| | 195 ± 5 | 0.2 | +3 ± 1 | |
| | 300 ± 8 | 0.16 | +10 ± 1 | |

### Example 3: Association of organic nanosystems to purified pollen particles

Either (i) 0.1 ml of the CS/TPP nanoparticles (0.4 mg/ml); or (ii) 0.5 ml of protamine nanocapsules (10 mg/ml) were added to 5 mg of the purified pollen particles of Example 1. The suspensions in each case were kept under horizontal stirring for 30 minutes at 37°C to thus obtain the platforms for administration according to the invention.

As an optional additional step, lyophilization of the platforms for 50 hours at -40°C (Labconco Corp.) which were then re-dispersed in a medium of interest, for example water or buffered water, was evaluated.

Both in the case of using lyophilization and in the case of applying only incubation, an effective association between the organic nanosystems of Example 2 and the purified pollen particle obtained in Example 1 was observed (SEM photograph, fluorescence measurements, and a SP5 Leica AOBS- SP5 confocal microscope). All this is done without the need to apply aggressive treatments such as acidic or basic media or the need to apply high pressures that may compromise capsule integrity and properties. Figure 8 shows SEM photographs of the pollen particles, prepared according to Example 1.1, which associate A) chitosan nanoparticles, B) protamine nanocapsules, and C) protamine nanocapsules after lyophilization. In the case of the samples subjected to lyophilization, an association and release study of the protamine nanosystems of Example 2 was conducted. A fluorescence study was conducted to evaluate the association, in which the amount of nanosystems not associated was determined at 0 min by means of centrifugation, and as a result an association efficiency over 60% of the nanosystems of Example 2.2 with the pollen was determined.

A release study was conducted with the nanosystems of Example 2.2 after a lyophilization process using as a fluorescent marker 5-TAMRA (5-carboxytetramethylrhodamine) covalently bound to protamine in order to obtain an effective labeling of the nanosystems. The release study was carried out by incubating the pollen with the nanosystems in water at 37ºC under orbital stirring. At given times up to a maximum of 4 hours, fluorescence quantifications were performed using the resulting supernatant after a centrifugation period of 2 minutes at 14000 rpm. The results indicate a sustained release of the nanosystems from the pollen. The gradual increase of the amount of nanosystems present in the medium was observed for at least 4 hours, obtaining at this point a release of about 80% with respect to the initial amount of nanosystems. See Figure 9.

### Example 4: Ex vivo evaluation of the purified pollen particles

### Preparation of the model

Male Sprague-Dawley rats with an approximate weight of 250 g were used. They were fasted for 18 hours before the experiment, leaving them only with access to water. The rats were sacrificed using CO₂ under suitable conditions in the hypoxia chamber. The intestines were immediately removed by means of an abdominal incision and washed with Krebs solution (pH 6.5) before preparing the sacs. This step is performed to eliminate food residues that may remain in the intestine. The intestine was divided into different parts. For administration, the samples were suspended in 1 ml of Krebs solution and introduced in the sacs with the help of a syringe. A sample was used to evaluate the anatomy and physiology of the intestine after treatment with hematoxylin-eosin, see Figure 10, and the rest was used for the analysis of the mucosal interaction capacity of the nanosystem complex and the biomaterial with the intestine by means of confocal microscopy.

### Results

The fact that the integrity of the intestine was maintained throughout the tests that were conducted stands out and indicates that the vehicles for administration of the invention are safe and do not cause side effects on tissue morphology.

After periods of incubating the purified pollen particles associated with organic nanosystems, intestinal tissue samples were observed by means of confocal microscopy. In all cases it was found that incubations lasting for 30 or 120 minutes lead to an improvement in the interaction and retention of the nanosystems on the intestinal mucosa. The confocal microscopy photographs taken at 120 minutes of Figures 11A, 11B, and 11C show how the nanoparticles are maintained on the purified pollen particles disclosed herein. It can be seen how the red column (Figure 10A), highlighting the nanoparticles, coincides with the green column (Figure 11B), highlighting the pollen particles. It can thus be seen how the nanosystems are kept deposited on the purified pollen particles disclosed herein.

In contrast, the administration of free nanosystems did not lead to the retention of appreciable amounts on the intestinal mucosa, and significant fluorescence by the nanoparticles in the tissue was not observed (as can be seen in the photographs referring to high concentration NCs and low concentration NCs), the effect of the adhesion promotion that is obtained when the purified pollen particles are combined with nanosystems thereby being found.

### Example 5: In vivo evaluation of the purified pollen particles

The experiment was performed with Sprague Dawley rats which were subjected to 12-hour fasting before carrying out the experiment.

The samples used consisted of 75 milligrams of pollen per 1 milliliter of water and were administered by means of oral gavage to each of the rats.

The observation was carried out after periods of 1 hour and 3 hours, respectively.

The rats were sacrificed by means of using a CO₂ chamber to remove the tissues. Then an incision was made in the abdomen of the animal through which different parts of the digestive tract were removed. These studies focus specifically on different parts of the small intestine (duodenum and jejunum).

After removing the tissues and without any type of treatment, direct observation was performed by means of an optical microscope at different magnifications.

The conclusions that were drawn from these experiments were that the pollen grains are stable under *in vivo* conditions because their retained characteristic morphology could be observed by means of microscopy, showing that the oral administration of the pollen *in vivo* can be effective because the pollen grains effortlessly reach different regions of the small intestine. See Figures 12 and 13. Tissue analysis by means of transverse sectioning allows concluding that the echinate morphology of the pollen allows for the efficient anchoring of pollen to the intestinal epithelium. Likewise, the histological evaluation (hematoxylin-eosin staining) indicates no pathological alteration derived from the administration and prolonged permanence of pollen in tissue.

## Claims

1. A delivery system comprising:
- a purified pollen particle comprising an intine layer and an exine layer; wherein said purified pollen particle does not comprise a lipid layer (*pollenkitt*); and
- a nanosystem; wherein the nanosystem is a lyophobic colloidal particle with a mean particle diameter comprised between 1 nm and 500 nm, wherein said mean diameter is measured by means of photon correlation spectroscopy .

2. The delivery system according to claim 1, wherein the purified pollen particle is a spinose purified pollen particle.

3. The delivery system according to any of the preceding claims; wherein the nanosystem is an organic colloidal particle; and
preferably wherein the polydispersity index of said mean particle diameter of the colloidal particle is comprised between 0.1 and 0.5, wherein said polydispersity index is measured by means of laser Doppler anemometry.

4. A method for the preparation of the delivery system as defined in any of claims 1 to 3, comprising the steps of:
(a) providing
- a pollen particle; and
- a nanosystem; wherein the nanosystem is a lyophobic colloidal particle with a mean particle diameter comprised between 1 nm and 500 nm, wherein said mean diameter is measured by means of photon correlation spectroscopy; and wherein the nanosystem is preferably an organic nanosystem;
(b) washing the pollen particle in an aqueous medium; preferably wherein the aqueous medium is a buffered aqueous medium at a pH comprised between 4.5 and 9;
(c) washing the pollen particle with an organic solvent;
wherein step (c) can be performed before or after step (b); to obtain a purified pollen particle comprising the intine layer and the exine layer; wherein the purified pollen particle does not comprises a lipid layer (*pollenkitt*); and
(d) contacting the purified pollen particle with the nanosystem to obtain a delivery system.

5. A delivery system obtainable by the method as defined in claim 4 comprising:
- a purified pollen particle comprising an intine layer and an exine layer; wherein said purified pollen particle does not comprises a lipid layer (*pollenkitt*); and
- a nanosystem; wherein the nanosystem is a lyophobic colloidal particle with a mean particle diameter comprised between 1 nm and 500 nm, wherein said mean diameter is measured by means of photon correlation spectroscopy.

6. The method according to claim 4, comprising an additional step of lyophilization.

7. A composition comprising the delivery system as defined in any of claims 1 to 3 and 5, wherein the delivery system, in particular the nanosystem of said delivery system, comprises a pharmaceutically acceptable active ingredient.

8. The composition as defined in claim 7 for use as a medicament.

9. The composition according to claim 8; wherein the delivery system is administrated by transmucosal administration; wherein the transmucosal administration is preferably oral administration, ocular administration, or nasal administration.

10. A composition comprising the delivery system as defined in any of claims 1 to 4 and 5, wherein the delivery system comprises a dietetically acceptable ingredient.

11. Use of the composition as defined in claim 10, for administering a dietetically acceptable ingredient; preferably for administering a flavor masking agent.

## Patentansprüche

1. Abgabesystem umfassend:
- ein gereinigtes Pollenpartikel, das eine Intin- und eine Exinschicht umfasst; wobei das gereinigte Pollenpartikel keine Lipidschicht (*Pollenkitt*) umfasst; und
- ein Nanosystem; wobei das Nanosystem ein lyophobes kolloidales Partikel mit einem mittleren Partikeldurchmesser umfassend zwischen 1 nm und 500 nm ist, wobei der mittlere Durchmesser mittels Photonenkorrelationsspektroskopie gemessen wird.

2. Abgabesystem nach Anspruch 1, wobei das gereinigte Pollenpartikel ein gereinigtes Spinose-Pollenpartikel ist.

3. Abgabesystem nach einem der vorangehenden Ansprüche, wobei das Nanosystem ein organisches kolloidales Partikel ist; und
bevorzugt wobei der Polydispersitätsindex des mittleren Partikeldurchmessers des kolloidalen Partikels zwischen 0,1 und 0,5 umfasst ist, wobei der Polydispersitätsindex mittels Laser-Doppler-Anemometrie gemessen wird.

4. Verfahren zur Herstellung eines wie in einem der Ansprüche 1 bis 3 definierten Abgabesystems, das die Schritte umfasst:
(a) Bereitstellen
- eines Pollenpartikels; und
- eines Nanosystems; wobei das Nanosystem ein lyophobes kolloidales Partikel mit einem mittleren Partikeldurchmesser umfassend zwischen 1 nm und 500 nm ist, wobei der mittlere Durchmesser mittels Photonenkorrelationsspektroskopie gemessen wird; und wobei das Nanosystem bevorzugt ein organisches Nanosystem ist;
(b) Waschen des Pollenpartikels in wässrigem Medium; bevorzugt wobei das wässrige Medium ein gepuffertes wässriges Medium bei einem pH-Wert ist, der zwischen 4,5 und 9 umfasst;
(c) Waschen des Pollenpartikels mit einem organischen Lösungsmittel;
- wobei Schritt (c) vor oder nach Schritt (b) durchgeführt werden kann, um ein gereinigtes Pollenpartikel zu erhalten, das die Intin- und die Exinschicht umfasst; wobei das gereinigte Pollenpartikel keine Lipidschicht (*Pollenkitt*) umfasst; und
(d) Inkontaktbringen des gereinigten Pollenpartikels mit dem Nanosystem, um ein Abgabesystem zu erhalten.

5. Abgabesystem, das mittels des in Anspruch 4 definierten Verfahren erhaltbar ist, umfassend:
- ein gereinigtes Pollenpartikel, dass eine Intin- und eine Exinschicht umfasst; wobei das gereinigte Pollenpartikel keine Lipidschicht (*Pollenkitt*) umfasst; und
- ein Nanosystem; wobei das Nanosystem ein lyophobes kolloidales Partikel mit einem mittleren Partikeldurchmesser umfassend zwischen 1 nm und 500 nm ist, wobei der mittlere Durchmesser mittels Photonenkorrelationsspektroskopie gemessen wird.

6. Verfahren nach Anspruch 4, das einen zusätzlichen Schritt einer Lyophilisation umfasst.

7. Zusammensetzung, die das wie in einem der Ansprüche 1 bis 3 und 5 definierte Abgabesystem umfasst, wobei das Abgabesystem, insbesondere das Nanosystem des Abgabesystems, einen pharmazeutisch verträglichen Wirkstoff umfasst.

8. Zusammensetzung wie in Anspruch 7 definiert zur Verwendung als Medikament.

9. Zusammensetzung nach Anspruch 8; wobei das Abgabesystem durch transmukosale Verabreichung verabreicht wird; wobei die transmukosale Verabreichung bevorzugt orale Verabreichung, okulare Verabreichung oder nasale Verabreichung ist.

10. Zusammensetzung, die das wie in einem der Ansprüche 1 bis 4 und 5 definierten Abgabesystem umfasst, wobei das Abgabesystem einen diätetisch verträglichen Bestandteil umfasst.

11. Verwendung einer wie in Anspruch 10 definierten Zusammensetzung zur Verabreichung eines diätetisch verträglichen Bestandteils; bevorzugt zur Verabreichung eines geschmacksmaskierenden Agenz.

## Revendications

1. Système d'administration comprenant :
- une particule de pollen purifié comprenant une couche intine et une couche exine ; dans lequel ladite particule de pollen purifié ne comprend pas de couche lipidique (*pollenkitt*) *;* et
- un nanosystème ; dans lequel le nanosystème est une particule colloïdale lyophobe ayant un diamètre moyen compris entre 1 nm et 500 nm, ledit diamètre moyen étant mesuré au moyen d'une spectroscopie de corrélation de photons.

2. Système d'administration selon la revendication 1, dans lequel la particule de pollen purifié est une particule de pollen épineux purifié.

3. Système d'administration selon l'une quelconque des revendications précédentes, dans lequel le nanosystème est une particule colloïdale organique ; et de préférence dans lequel l'indice de polydispersité dudit diamètre moyen des particules colloïdales est compris entre 0,1 et 0,5, ledit indice de polydispersité étant mesuré au moyen d'une anémométrie laser Doppler.

4. Procédé de préparation du système d'administration tel que défini dans l'une quelconque des revendications 1 à 3, comprenant les étapes suivantes :
(a) fournir
- une particule de pollen ; et
- un nanosystème ; dans lequel le nanosystème est une particule colloïdale lyophobe ayant un diamètre moyen compris entre 1 nm et 500 nm, ledit diamètre moyen étant mesuré au moyen d'une spectroscopie par corrélation de photons ; et dans lequel le nanosystème est de préférence un nanosystème organique ;
(b) laver la particule de pollen dans un milieu aqueux ; de préférence, le milieu aqueux étant un milieu aqueux tamponné à un pH compris entre 4,5 et 9 ;
(c) laver la particule de pollen avec un solvant organique ;
dans lequel l'étape (c) peut être réalisée avant ou après l'étape (b) ; pour obtenir une particule de pollen purifié comprenant la couche intine et la couche exine ; dans laquelle la particule de pollen purifié ne comprend pas de couche lipidique (*pollenkitt*) *;* et
(d) mettre en contact la particule de pollen purifié avec le nanosystème pour obtenir un système d'administration.

5. Système d'administration pouvant être obtenu par le procédé défini dans la revendication 4, comprenant :
- une particule de pollen purifié comprenant une couche intine et une couche exine ; dans laquelle ladite particule de pollen purifié ne comprend pas de couche lipidique (pollenkitt) ; et
- un nanosystème ; dans lequel le nanosystème est une particule colloïdale lyophobe ayant un diamètre moyen compris entre 1 nm et 500 nm, dans lequel ledit diamètre moyen est mesuré au moyen d'une spectroscopie de corrélation de photons.

6. Procédé selon la revendication 4, comprenant une étape supplémentaire de lyophilisation.

7. Composition comprenant le système d'administration tel que défini dans l'une quelconque des revendications 1 à 3 et 5, dans laquelle le système d'administration, en particulier le nanosystème dudit système d'administration, comprend un ingrédient actif pharmaceutiquement acceptable.

8. Composition telle que définie dans la revendication 7 pour une utilisation en tant que médicament.

9. Composition selon la revendication 8, dans laquelle le système d'administration est administré par voie transmucosale, l'administration transmucosale étant de préférence une administration orale, une administration oculaire ou une administration nasale.

10. Composition comprenant le système d'administration tel que défini dans l'une quelconque des revendications 1 à 4 et 5, dans laquelle le système d'administration comprend un ingrédient acceptable sur le plan diététique.

11. Utilisation de la composition telle que définie dans la revendication 10, pour administrer un ingrédient acceptable sur le plan diététique ; de préférence pour administrer un agent masquant le goût.
